# EUROPEAN PATENT APPLICATION

(11) **EP 4 361 245 A1**
(43) Date of publication of application: **01.05.2024**
(21) Application number: 22828694.4
(22) Date of filing: 20.06.2022
(51) Int. Cl.: C12M 1/34, C12M 1/00

(54) **CELL THAWING DEVICE**

(30) Priority: 25.06.2021 KR 20210082930
(71) Applicant: Amogreentech Co., Ltd., Gimpo-si, Gyeonggi-do 10014 (KR); Kangstem Biotech Co., Ltd., Seoul 06179 (KR)
(72) Inventor: HAN, Kyoung Ku, Gimpo-si, Gyeonggi-do 10014 (KR); SONG, Jae Kyung, Gimpo-si, Gyeonggi-do 10014 (KR); JANG, Seon Ho, Gimpo-si, Gyeonggi-do 10014 (KR); SEO, In Yong, Gimpo-si, Gyeonggi-do 10014 (KR); KIM, Jae Yun, Gimpo-si, Gyeonggi-do 10014 (KR); KANG, Kyung Sun, Seoul 06338 (KR); LEE, Mi Hye, Seoul 08861 (KR); HEO, Hyun Suk, Daegu 41226 (KR)
(74) Representative: Treeby, Philip David William
(86) International application number: PCT/KR2022/008714
(87) International publication number: WO 2022/270847

(57) **Abstract**

A cell thawing device is provided. A cell thawing device according to one embodiment of the present invention is a cell thawing device for thawing biological material comprising cells, the cell thawing device comprising: a housing; a body part which is disposed inside the housing and may form an accommodation space so as to surround at least a portion of a container in which the biological material is stored; a moving part for moving the body part toward the container so that the body part forms the accommodation space; a heating part which is installed in the body part and transfers heat to the container disposed in the accommodation space; and a sensing part for detecting a physical quantity of the body part or the container, wherein a heat transfer space is formed between the body part and the container such that heat can be transferred from the body part to the container in a non-contact manner.

## Description

### [Technical Field]

The present invention relates to a cell thawing device.

### [Background Art]

In general, various biological materials are stored in a container such as a vial for long-term storage, and then stored at a low temperature.

As an example, plasma and tissue cells are stored at a maximum of -100 degrees Celsius, and stem cells are stored at a cryogenic temperature of up to -165 degrees Celsius by using a liquid nitrogen gas phase.

On the other hand, the experiments and usage of biological materials are performed in an environment whose temperature is higher than the storage temperature of the aforementioned low-temperature state. Therefore, in order to use the low-temperature biological material, the process of heating the same to thaw is required.

To this end, there has been proposed a cell thawing device that thaws a biological material by accommodating a container such as a vial and then heating the same under a certain condition.

However, in the case of conventionally proposed cell thawing devices, the method of transferring heat in a state of direct contact with a container has been adopted. Therefore, in the case of such cell thawing devices, there is a problem in that it is difficult to uniformly heat the container as a whole, and there is also a problem in that the container may be cracked or deformed when a high-temperature heating element directly contacts the container.

Considering this situation, it is urgent to develop a cell thawing device which is capable of uniformly heating the container as a whole and stably protecting the container during heating.

### [Disclosure]

### [Technical Problem]

The present invention has been devised in view of the above points, and an object of the present invention is to provide a cell thawing device which is capable of uniformly heating the container as a whole while ensuring the stability of a container when heating the container including a biological material.

In addition, another object of the present invention is to provide a cell thawing device which is capable of effectively heating a container while increasing the efficiency of energy usage.

### [Technical Solution]

In order to achieve the above-described objects, the present invention provides a cell thawing device for thawing a biological material including cells, the cell thawing device including a housing; a body part which is disposed inside the housing and may form an accommodation space so as to surround at least a portion of a container in which the biological material is stored; a moving part for moving the body part toward the container such that the body part forms the accommodation space; a heating part which is installed in the body part and transfers heat to the container disposed in the accommodation space; and a sensing part for detecting a physical quantity of the body part or the container, wherein a heat transfer space is formed between the body part and the container such that heat can be transferred from the body part to the container in a non-contact manner.

In addition, the accommodation space may be formed in a cylindrical shape to correspond to the shape of the container, and the diameter of the accommodation space may be formed to be larger than the diameter of the container by a predetermined width.

In this case, the predetermined width may be 0.2 mm to 0.3 mm.

In addition, in the body part, a protruding member may be formed on an upper portion of an opposing surface facing the container so as to protrude up to an outer peripheral surface of the container along the circumferential direction of the opposing surface.

In addition, the body part may include a first body which surrounds one side portion of the container; and a second body which surrounds the other side portion of the container and forms the accommodation space together with the first body.

In addition, the moving part may include a rotation shaft; a moving member which is movably coupled to the rotation shaft; a first link member which connects one side of the moving member and the first body; and a second link member which connects the other side of the moving member and the second body, wherein as the moving member moves, the first body and the second body may be moved away from or closer to the container in the accommodation space.

In addition, the heating part may include a first heating part which is disposed on an outer side of the first body opposite to an opposing surface facing the container; and a second heating part which is disposed in the second body and arranged to be symmetrical to the first heating part with respect to the container.

In addition, the heating part may include a heater which is in contact with the body part through a contact surface; and a heat insulating member which surrounds an outer side of the heater excluding the contact surface such that heat generated from the heater is transferred in a direction toward the container.

In addition, the heater may include a heating element which generates heat when power is applied; and a support body which is made of a ceramic material and surrounds at least a portion of the heating element.

In addition, the sensor part may include a non-contact sensor that is disposed at a predetermined distance from the body part and provides information on whether the container is placed in the accommodation space.

In addition, the non-contact sensor may be an infrared sensor.

In addition, the cell thawing device may further include a control part which comprehensively controls the operations of the moving part, the heating part and the sensor part, wherein the control unit may be configured not to apply a control signal to operate the moving part or the heating part, when it is determined that the container is not present in the accommodation space based on information transmitted from the sensor part.

### [Advantageous Effects]

According to the present invention, by heating a container which is disposed in the accommodation space formed by the body part through a non-contact heating manner using convection and radiation, the container can be uniformly heated along the longitudinal direction.

The present invention can prevent the cracks or thermal deformation of a container that may occur during heat conduction through contact by introducing the above-described non-contact heating manner.

In addition, the present invention can indirectly heat the container through a heating part, thereby preserving the heat of the body part for a certain period of time. As a result, the present invention can improve the efficiency of energy usage during the continuous heating of a plurality of containers.

### [Description of Drawings]

FIG. 1 is a perspective view showing a cell thawing device according to one embodiment of the present invention;
FIG. 2 is a perspective view showing a body part, a moving part, a heating part and a sensor part of the cell thawing device according to one embodiment of the present invention;
FIG. 3 is a separation view of FIG. 2;
FIG. 4 is a perspective view showing the formation of an accommodation space by the body part in FIG. 2;
FIGS. 5 and 6 are cross-sectional views of FIG. 2;
FIG. 7 is a view showing a heating part of the cell thawing device according to one embodiment of the present invention;
FIG. 8 is a side view of FIG. 2; and
FIG. 9 is a block diagram for explaining the integrated control of a control part of the cell thawing device according to one embodiment of the present invention.

### [Modes of the Invention]

Hereinafter, with reference to the accompanying drawings, the exemplary embodiments of the present invention will be described in detail so that those skilled in the art can easily practice the present invention. The present invention may be embodied in many different forms and is not limited to the exemplary embodiments set forth herein. In order to clearly describe the present invention in the drawings, parts that are irrelevant to the description are omitted, and the same reference numerals are assigned to the same or similar components throughout the specification. In addition, the size or shape of the components illustrated in the drawings may be exaggerated for the clarity and convenience of description.

The cell thawing device 100 according to one embodiment of the present invention is a device for heating and thawing a biological material that is maintained in a low temperature state (e.g., frozen state). Herein, the biological material may include cells, which are the structural basic unit of an organism.

In this case, in terms of heating a container 10 (hereinafter, referred to as a 'container') in which the biological material is stored, the cell thawing device 100 according to one embodiment of the present invention may heat the container in a non-contact manner in order to heat the container 10 more stably and effectively. Herein, the container 10 may be a known experimental/medical container such as a vial, a beaker or a test tube.

To this end, the cell thawing device 100 according to one embodiment of the present invention may include a housing 110, a body part 120, a moving part 130, a heating part 140, a sensor part 150 and a control part 160.

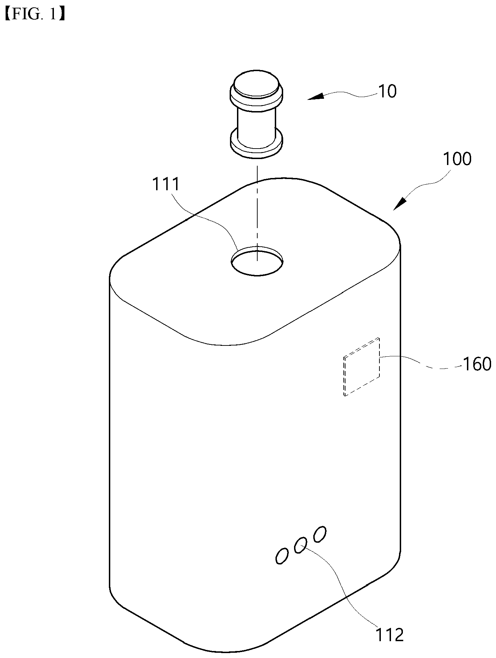

First of all, referring to FIG. 1, the housing 110 forms an outer surface of the cell thawing device 100, and it may include a space such that the body part 120, the moving part 130, the heating part 140, the sensor part 150 and the control part 160 to be described below may be disposed therein.

In this case, an opening 111 having a predetermined diameter may be formed in an upper portion of the housing 110 such that the container 10 may be inserted into the housing 110. Herein, the opening 111 may be formed in a circular shape as illustrated in the drawings to correspond to the container 10 formed in a cylindrical shape. As a result, when the container 10 is inserted through the opening 111, only a cap part 11 of the container 10 may be exposed from the outside.

In addition, a manipulation part 112 for manipulating the operation of the cell thawing device 100 according to one embodiment of the present invention may be provided outside the housing 110.

In this case, the manipulation part 112 is a medium for transmitting a command to the control part 160 to be described below, and it may be, for example, a pressurized physical button or a capacitive touch button. Through this, the user can transmit, for example, commands related to operation and stop of the cell thawing device 100, heating temperature control and the like to the control part 160.

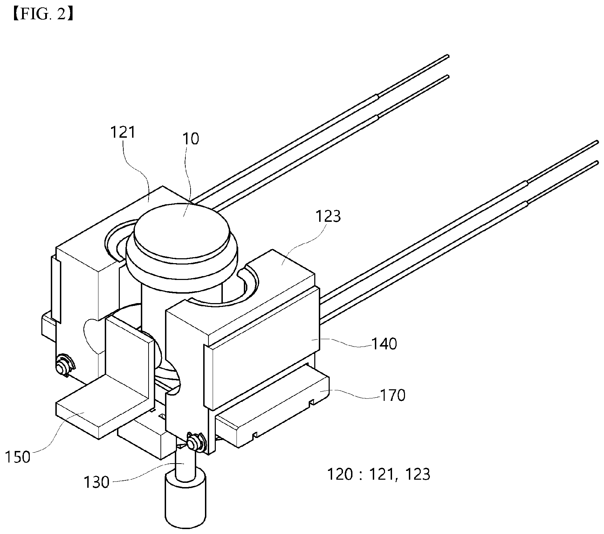

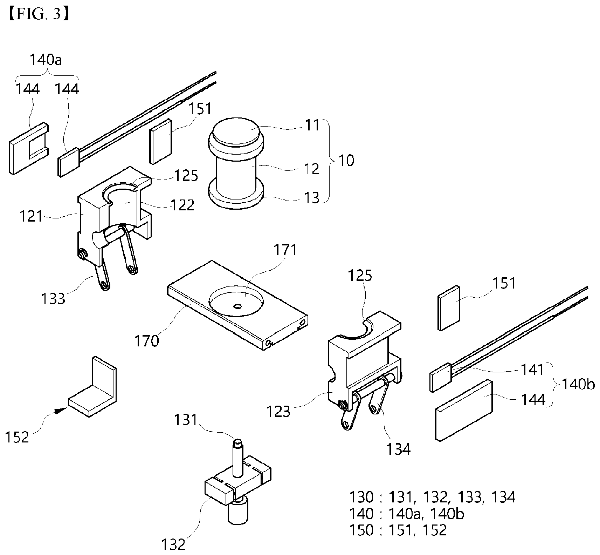

Next, referring to FIGS. 2 and 3, the cell thawing device 100 according to one embodiment of the present invention may include a body part 120 for forming an accommodation space S1 inside the housing 110 so as to surround part or all of the container 10 described above.

As a specific example, the body part 120 may include a first body 121 and a second body 123 that are respectively disposed on both sides of the container 10.

In this case, as illustrated in FIG. 3, the first body 121 and the second body 123 may have opposing surfaces 122, 124 that are curved inward to correspond to the shape of an outer circumferential part 12 of the container 10 formed in a cylindrical shape. That is, the opposing surfaces 122, 124 may be formed by including curved surfaces obtained by cutting a cylinder in a vertical direction.

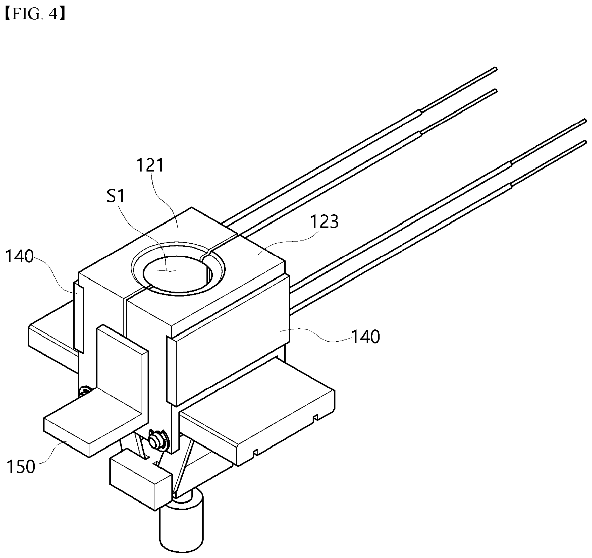

Through this, when the first body 121 and the second body 123 come into contact with each other as illustrated in FIG. 4, the opposing surfaces 122, 124 which are respectively formed on the first body 121 and the second body 123 may be connected to each other to form an accommodation space S 1 in a cylindrical form so as to sufficiently surround an outer side of the container 10.

However, although the body part 120 is illustrated as including only the first body 121 and the second body 123 in the drawings, the example of the body part 120 of the cell thawing device 100 according to one embodiment of the present invention is not limited thereto.

In other words, the body part 120 is formed as a single entity, but may be formed so as to be able to adjust the diameter, or in addition to the first body 121 and the second body 123, it may be formed by further including a third body (not illustrated) or a fourth body (not illustrated).

In one embodiment of the present invention, the body part 120 may be formed of a material having excellent thermal conductivity, such as metal.

Through this, the heat transferred to the body part 120 by the heating part 140, which will be described below, is finally transferred to the opposing surfaces 122, 124 via the body part 120 such that the container 10 adjacent to the opposing surfaces 122, 124 may be heated.

In addition, the body part 120 may be formed to have a heat capacity that is higher than a certain level. This allows the body part 120 to keep the heat transferred from the heating part 140 inside for a certain period of time rather than immediately radiating the same to the outside, and thus, when a plurality of containers are sequentially heated, the time required for initial heating is minimized to enhance the efficiency of energy consumption.

Meanwhile, in one embodiment of the present invention, the opposing surfaces 122, 124 forming the accommodation space S1 and the outer circumferential part 12 of the container 10 maintain a non-contact state in which they do not come into contact with each other, and accordingly, non-contact heat transfer may be formed from the body part 120 to the side of the container 10.

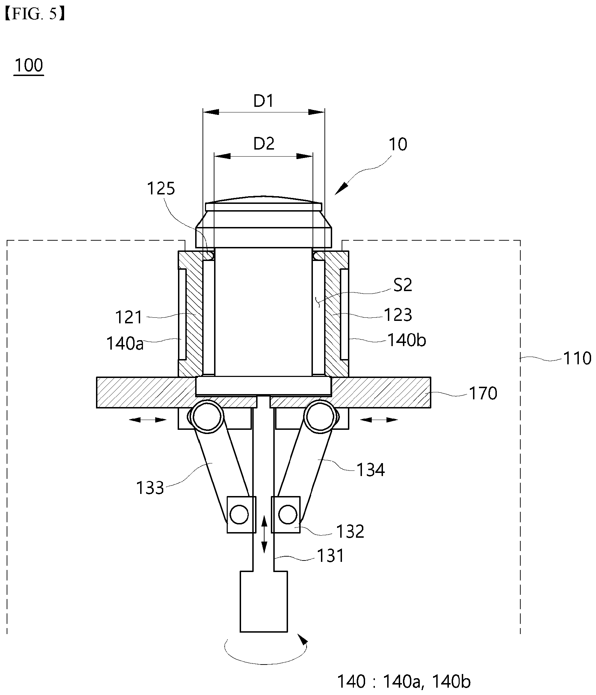

Hereinafter, the non-contact heating manner of the cell thawing device 100 according to one embodiment of the present invention will be described in more detail with reference to FIG. 5.

As illustrated in FIG. 5, when the first body 121 and the second body 123 are adjacent to the container 10 to form the accommodation space S1, a heat transfer space S2 having a predetermined volume may be formed between the opposing surfaces 122, 124 of the first body 121 and the second body 123 and the outer circumferential part 12 of the container. Herein, the heat transfer space S2 may be an outer part of the above-described accommodation space S 1.

In this case, the reason that the heat transfer space S2 may be formed is that as illustrated in FIG. 5, the diameter D1 of the accommodation space S1 formed by the first body 121 and the second body 123 is formed to be greater than the diameter D2 of the outer circumferential part 12 of the container by predetermined widths (D1-D2).

By forming the heat transfer space S2 in this way, heat may be transferred from the high-temperature body part 120 to the side of the container 10 in a relatively low-temperature state through convection and radiation (instead of conduction).

As such, the cell thawing device 100 according to one embodiment of the present invention may transfer heat uniformly along the longitudinal direction of the container 10, by using heat transfer through convection and radiation. That is, it is possible to prevent heat transfer from being biased to a specific area of the container 10 compared to a case in which a high-temperature body part or a heater is directly contacted to transfer heat.

Moreover, it is possible to prevent cracks or thermal deformation that may occur when the body part or the heater directly contacts the outer circumferential part 12 of the container, which is made of glass or plastic and has low strength.

In one embodiment of the present invention, the predetermined widths (D1-D2) formed between the opposing surfaces 122, 124 of the body part 120 and the outer circumferential part 12 of the container may be 0.2 mm to 0.3 mm. These numerical values have been derived through repeated experiments, and these numerical values have been derived by comprehensively considering that if the heat transfer space is too large, excessive energy is required, and conversely, if the heat transfer space is too small, heat transfer through convection and radiation may be minimal.

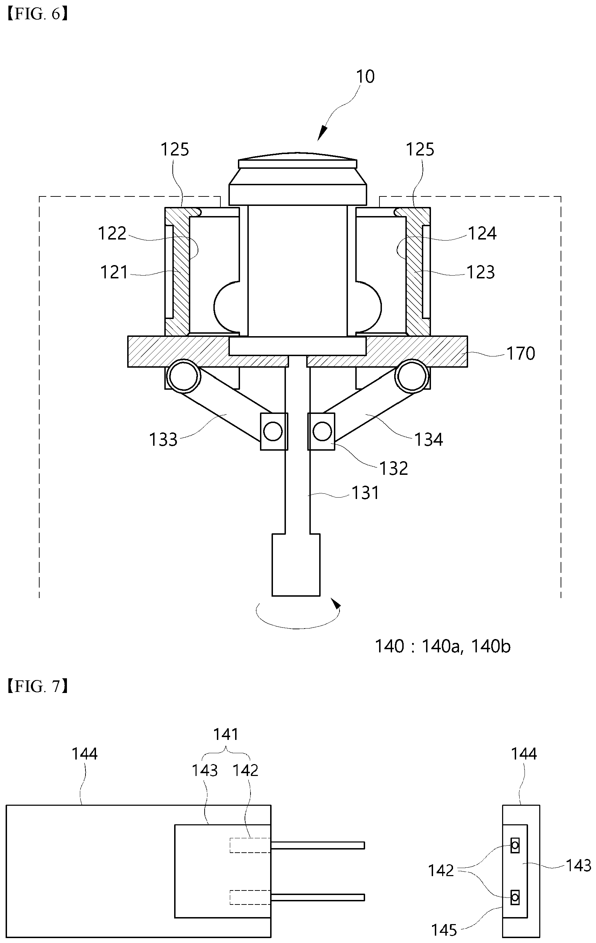

Referring to FIGS. 5 and 6, the body part 120, for example, the upper portion of the opposing surfaces 122, 124 facing the container in the first body 121 and the second body 123 of the container 10 may be formed with a protruding member 125 that can locally contact a portion of the outer circumferential part 12 of the container 10.

In this case, the protruding member 125 may be formed to protrude from the opposite surfaces 122, 124 in a direction of the container 10, and it may have a ring shape by being continuously formed along the circumferential direction of the outer circumferential part 12 of the container.

As such, the cell thawing device 100 according to one embodiment of the present invention may spatially block the upper region of the heat transfer space S2 through the protruding member 125. Through this, by minimizing the heat flowing outward from the heat transfer space S2 to the outer side of the body part 120, the heat transfer efficiency may be maximized.

Meanwhile, the protruding member 125 may be formed of a material having elasticity in order to mitigate impact upon contact with the outer circumferential part 12 of the container and maximize the contact area. However, the material of the protruding member 125 is not limited thereto, and the protruding member 125 may be formed of the same material as the body part 120, by being integrally formed with the body part 120 in consideration of the convenience of fabrication.

The cell thawing device 100 according to one embodiment of the present invention may include a base part 170 on which the container 10 can be seated.

In this case, as illustrated in FIG. 3, in the center of the base part 170, a seating groove 171 having an area corresponding to a flange part 13 that is formed in a lower portion of the container 10 may be formed to maintain a stable state when the container 10 is seated. By inserting the flange part 13 of the container 10 into the seating groove 171, the container 10 may maintain an upright state without tilting in any one direction.

In addition, although not illustrated in the drawings, the center of the base part 170 may be formed with a seating protrusion (not illustrated) which is formed to protrude in a direction toward a flange groove (not illustrated) so as to be inserted into the flange groove that is formed on a lower surface of the flange part 13.

Moreover, in one embodiment of the present invention, when the first body 121 and the second body 123 are moved by the moving part 130, the base part 170 may function as a guide rail to guide the movement of the first body 121 and the second body 123. That is, when an external force is applied from the moving part 130 while the first body 121 and the second body 123 are movably coupled to the base part 170, it may be moved along the longitudinal direction of the base part 170.

Referring to FIGS. 3 to 6, the cell thawing device 100 according to one embodiment of the present invention may include a moving part 130 for moving the body part 120 described above.

Specifically, before the container 10 is inserted into the housing 110, the body part 120 may be disposed in a state of being spaced apart from each other around the seating groove 171 in which the container 10 is seated, as illustrated in FIG. 2. This is to allow the container 10 to enter the inside of the housing 110 freely without being affected by the first body 121 and the second body 123, when the container 10 is inserted into the inside of the housing 110.

Afterwards, when the container 10 is inserted into the housing 110, the first body 121 and the second body 123 form the above-described accommodation space S1, and in order to limit the heating range, as illustrated in FIG. 5, it may be moved closer to the container 10 by the moving part 130.

As a non-limiting example, such a moving part 130 may be formed by including a rotating shaft 131, a moving member 132, a first link member 133 and a second link member 134 as illustrated in FIGS. 5 and 6.

In this case, the rotating shaft 131 may be a member that is capable of rotating in a clockwise or counterclockwise direction around a direction perpendicular to the ground, and for example, it may be directly connected to an electric motor to perform the above-described shaft rotation.

In this regard, the movable member 132 may be movably coupled to an outer side of the rotation shaft 131, and may rise or fall in a direction perpendicular to the ground when the rotation shaft 131 rotates axially.

In this case, in order to convert a rotational motion of the rotating shaft 131 into a linear motion of the moving member 132, for example, the outer circumferential surface of the rotating shaft 131 and the inner circumferential surface of the movable member 132 may respectively be formed with a screw thread (not illustrated) that is formed to engage with each other. However, the above-described example is only an example of the rotating shaft 131 and the moving member 132, and other various structures that are capable of converting rotational motion into linear motion, such as a ball screw structure, may be applied.

Meanwhile, one end portion of each of the first link member 133 and the second link member 134 may be rotatably connected to both sides of the movable member 132. In addition, the other end portions of the first link member 133 and the second link member 134 may be rotatably connected to the first body 121 and the second body 123, respectively.

Accordingly, as illustrated in FIG. 5, when the moving member 132 is raised or lowered by the axis rotation of the rotating shaft 131, as the first link member 133 and the second link member 134 connected to the moving member 132 are moved in a direction away from or closer to the rotating shaft 131, the first body 121 and the second body 123 connected thereto may also move away from or closer to the container 10 located above the rotating shaft 131.

In this case, the first link member 133 and the second link member 134 have the same length such that the first body 121 and the second body 123 may be symmetrically moved to each other with respect to the container 10. As a result, when the first body 121 and the second body 123 come into contact to form the accommodation space S1, it is possible to form the accommodation space S1 with high airtightness without tolerance.

As described above, while the cell thawing device 100 according to one embodiment of the present invention is provided with a moving part 130 having a relatively simple structure, it has the advantage in that the first body 121 and the second body 123 may form left-right symmetry with high precision.

Meanwhile, the detailed configuration and movement mechanism of the moving part 130 described above is only an example of the moving part 130, and the application of the cell thawing device 100 according to one embodiment of the present invention is not limited thereto. That is, other than the above-described examples, various structures of the moving part 130 may be applied.

The cell thawing device 100 according to one embodiment of the present invention may include a heating part 140 that transfers heat to the body part 120.

In this case, the heating part 140 is a member that is capable of converting electrical energy into heat, and as illustrated in FIGS. 4 and 5, it may be installed on one side of the body part 120 so as to be in contact with the body part 120. Through this, the heating part 140 may transfer heat to the side of the body part 120 having thermal conductivity by using a conduction phenomenon.

In one embodiment of the present invention, the heating part 140 may include a first heating part 140a which is installed on the first body 121 and a second heating part 140b which is installed on the second body 123.

In this case, referring to FIGS. 5 and 6, the first heating part 140a and the second heating part 140b may be disposed parallel to the longitudinal direction of the container 10 on the outer side opposite to the opposing surfaces 122, 124 facing the container, among the first body 121 and the second body 123, respectively.

Through this, the heating part 140 may transfer heat uniformly without being biased to any one side with respect to the longitudinal direction of the container 10 in which the biological material is stored.

In addition, the first heating part 140a and the second heating part 140a may be disposed symmetrically with respect to the container 10. As a result, the first heating part 140a and the second heating part 140b may be heated to have the same or similar heat distribution between the first body 121 and the second body 123 that are disposed on both sides of the container 10.

In one embodiment of the present invention, referring to FIG. 7, the heating part 140 may include a heater 141 and a heat insulating member 144.

Specifically, the heater 141 may directly contact the first body 121 or the second body 123 through a contact surface to transfer heat toward the body part 120.

In addition, the heat insulating member 144 may be formed of a material having heat insulating properties, and as illustrated in FIG. 7, it is arranged to surround an outer side of the heater 141 except for the contact surface 145, thereby preventing the heat generated from the heater 141 from leaking out in directions other than the body part 120.

Through this, the cell thawing device 100 according to one embodiment of the present invention may induce the heat generated from the heater 141 to be intensively transferred only in a direction toward the container 10, that is, toward the body part 120. As a result, it is possible to improve the consumption efficiency of thermal energy, and it is possible to suppress an abnormal increase in the temperature of the inner space of the housing 110 such that mechanical stability may be secured.

Meanwhile, as a non-limiting example, the heater 141 may be formed of a ceramic heater.

More specifically, the heater 141 may include a heating element 142 which is formed of a resistor having a predetermined resistance and generating heat when power is applied, and a support body 143 surrounding the heating element 142.

In this case, the support body 143 may be made of a ceramic material such as, for example, alumina, ZrO₂, MgO, Si₃N₄, SiC, AlN, ZTA and the like. Through this, the cell thawing device 100 according to one embodiment of the present invention may secure reliability even under operating conditions where heating and cooling are repeatedly performed, and may improve the lifespan cycle of the product.

In addition, as illustrated in the drawings, the support body 143 has a predetermined area and is arranged to surround the heating element 142, thereby expanding the transfer area of heat diffused from the heating element 142. As a result, since the heating area can be increased with respect to the body part 120 having a large area compared to the heating element 142, there is an advantage in that the body part 120 may be uniformly heated as a whole.

However, the type of the heater 141 is not limited to the above-described ceramic heater, and various known heaters may be applied as needed.

Meanwhile, although the drawings illustrated that the heating part 140 is installed outside the body part 120, according to the design, the heating part 140 may be installed inside the body part 120 so as not to be observed from the outside.

The cell thawing device 100 according to one embodiment of the present invention may include a sensor part 150 for sensing the physical quantity of the above-described body part 120 or container 10. Herein, the physical quantity may be at least one of temperature, humidity, weight and distance.

In this case, the sensor part 150 may include a first sensor 151 and a second sensor 152.

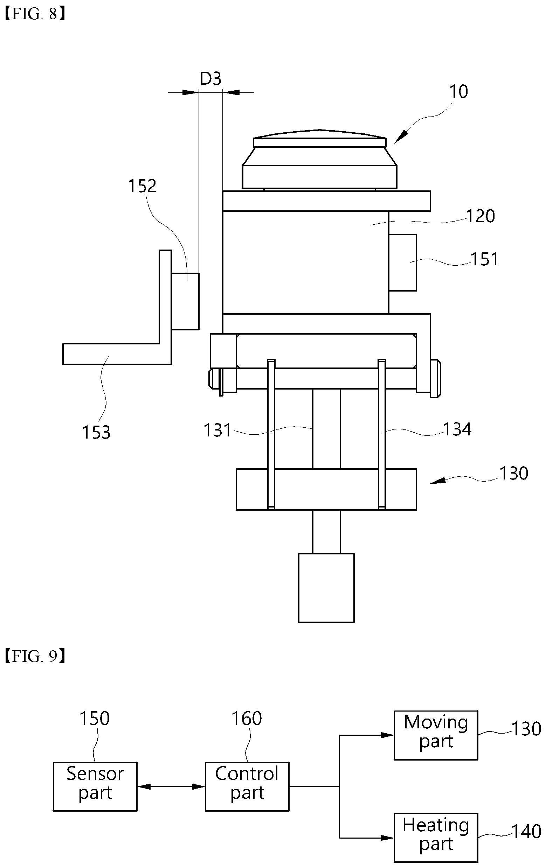

First of all, the first sensor 151 is for sensing the temperature of the first body 121 or the second body 123, and as illustrated in FIG. 8, it may be a contact sensor installed in direct contact with the first body 121 or the second body 123.

As a non-limiting example, the first sensor 151 may be formed of a known contact type sensor such as a thermocouple, a resistance thermometer (RTD) or a thermistor.

As such, the first sensor 151 may more precisely measure the temperature through direct contact with the body part 120, and may transmit the measured temperature information to the control part 160 to be described below.

Next, referring again to FIG. 8, the second sensor 152 may be a non-contact sensor that is disposed to be spaced apart from the body part 120 unlike the first sensor 151.

In this case, the second sensor 152 may detect whether the container 10 is located inside the body part 120. This is to prevent the body part 120 from overheating due to an error in an operation command even when the container 10 is not disposed within the housing 110.

As an illustrative example, the second sensor 152 may be formed of an infrared sensor (IR sensor). In this case, the infrared sensor detects a minute temperature change that occurs when the container 10 is located on the body part 120 even when the container 10 is spaced apart from the body part 120 such that it is possible to detect whether the container 10 is located inside the body part 120.

That is, in the cell thawing device 100 according to one embodiment of the present invention, the heating part is not located in a region adjacent to the outer circumference 12 of the container 10, but as described above, the heating part 140 is located at a relatively far distance from the opposing surfaces 122, 124 adjacent to the container 10 such that the infrared sensor may not be affected by noise caused by the initial heating of the heating part 140. Accordingly, although the infrared sensor is a non-contact sensor, it may relatively detect accurately whether the container 10 has entered.

However, the second sensor 152 may be various known non-contact sensors such as an ultrasonic sensor and a magnetic sensor, in addition to the above-described infrared sensor.

Meanwhile, the second sensor 152 may be located in a state of being installed in a separate fixture 153 at a distance adjacent to the body part 120 so as to be spaced apart from the body part 120 by a predetermined distance D3.

In this way, the reason why the second sensor 152 is disposed to be spaced apart from the body part 120 is that when the second sensor 152 is installed in the body part 120 to measure a physical quantity related to the container 10, it may lower the airtightness of the accommodation space S 1.

Moreover, when the sensor is disposed to be spaced apart from the body part 120 like the second sensor 152, it is possible to prevent the durability of the sensor from deteriorating by a repeated temperature increase inside the accommodation space S1 even if a separate heat insulating member is not added to the sensor.

The cell thawing device 100 according to one embodiment of the present invention may include a control part 160 that integrally controls the operations of the moving part 130, the heating part 140 and the sensor part 150 described above.

In this case, the control part 160 may control the moving part 130, the heating part 140 and the sensor part 150 based on a user's command transmitted from the manipulation part 112.

In addition, the control part 160 may perform the determination by itself.

As a specific example, referring to FIG. 9, if the control part 160 determines that the container 10 is absent in the body part 120 based on the information transmitted from the second sensor 152, it may not apply a control signal for operating the moving part 130 or the heating part 140.

Conversely, when it is determined that the container 10 is located within the body part 120, the control part 160 may apply a control signal to operate the moving part 130 or the heating part 140 by a user's command or automatically.

Through this, it is possible to prevent energy from being wasted due to overheating of the body part 120 by the heating part 140 in the absence of the container 10. Particularly, for example, before product shipment, repetitive test operations are required to check the performance of the product, and in this case, only when the container 10 is present in the accommodation space S1 by the control part 160 described above, it is possible to improve the convenience of work by automatically operating the cell thawing device 100.

Although one exemplary embodiment of the present invention has been described above, the spirit of the present invention is not limited to the exemplary embodiments presented herein, and those skilled in the art who understand the spirit of the present invention may easily suggest other exemplary embodiments by changing, modifying, deleting or adding components within the scope of the same spirit, but this will also fall within the scope of the present invention.

## Claims

1. A cell thawing device for thawing a biological material including cells, the cell thawing device comprising:
a housing;
a body part which is disposed inside the housing and may form an accommodation space so as to surround at least a portion of a container in which the biological material is stored;
a moving part for moving the body part toward the container such that the body part forms the accommodation space;
a heating part which is installed in the body part and transfers heat to the container disposed in the accommodation space; and
a sensing part for detecting a physical quantity of the body part or the container,
wherein a heat transfer space is formed between the body part and the container such that heat can be transferred from the body part to the container in a non-contact manner.

2. The cell thawing device of claim 1, wherein the accommodation space is formed in a cylindrical shape to correspond to the shape of the container, and
wherein the diameter of the accommodation space is formed to be larger than the diameter of the container by a predetermined width.

3. The cell thawing device of claim 2, wherein the predetermined width is 0.2 mm to 0.3 mm.

4. The cell thawing device of claim 2, wherein in the body part, a protruding member is formed on an upper portion of an opposing surface facing the container so as to protrude up to an outer peripheral surface of the container along the circumferential direction of the opposing surface.

5. The cell thawing device of claim 1, wherein the body part comprises:
a first body which surrounds one side portion of the container; and
a second body which surrounds the other side portion of the container and forms the accommodation space together with the first body.

6. The cell thawing device of claim 5, wherein the moving part comprises:
a rotation shaft;
a moving member which is movably coupled to the rotation shaft;
a first link member which connects one side of the moving member and the first body; and
a second link member which connects the other side of the moving member and the second body,
wherein as the moving member moves, the first body and the second body may be moved away from or closer to the container in the accommodation space.

7. The cell thawing device of claim 5, wherein the heating part comprises:
a first heating part which is disposed on an outer side of the first body opposite to an opposing surface facing the container; and
a second heating part which is disposed in the second body and arranged to be symmetrical to the first heating part with respect to the container.

8. The cell thawing device of claim 1, wherein the heating part comprises:
a heater which is in contact with the body part through a contact surface; and
a heat insulating member which surrounds an outer side of the heater excluding the contact surface such that heat generated from the heater is transferred in a direction toward the container.

9. The cell thawing device of claim 8, wherein the heater comprises:
a heating element which generates heat when power is applied; and
a support body which is made of a ceramic material and surrounds at least a portion of the heating element.

10. The cell thawing device of claim 1, wherein the sensor part comprises a non-contact sensor that is disposed at a predetermined distance from the body part and provides information on whether the container is placed in the accommodation space.

11. The cell thawing device of claim 10, wherein the non-contact sensor is an infrared sensor.

12. The cell thawing device of claim 1, further comprising:
a control part which comprehensively controls the operations of the moving part, the heating part and the sensor part,
wherein the control unit is configured not to apply a control signal to operate the moving part or the heating part, when it is determined that the container is not present in the accommodation space based on information transmitted from the sensor part.
